# EUROPEAN PATENT APPLICATION

(11) **EP 4 850 633 A1**
(43) Date of publication of application: **09.12.2026**
(21) Application number: 25305824.2
(22) Date of filing: 05.06.2025
(51) Int. Cl.: A61F 2/00, D04B 21/12, D04D 9/04

(54) **METHOD OF MANUFACTURING A SELF-GRIPPING SURGICAL MESH**

(71) Applicant: Sofradim Production, 01600 Trevoux (FR)
(72) Inventor: MIRA, Anthony, 01600 TREVOUX (FR); FUMET, Nicolas, 01600 TREVOUX (FR)
(74) Representative: Germain Maureau

(57) **Abstract**

The present technology is generally related to self-fixation material for use in hernia repair surgical mesh. The present technology is directed to a method for forming a surgical mesh (300) having one or more barbs. The method includes forming at least one looped strip (302) within a mesh sheet, processing the mesh sheet to convert one or more of the plurality of loops (316) into the one or more barbs, removing at least the first temporary filament (312A) and the second temporary filament (312B) to form at least one fixation ribbon (320) having the one or more barbs (310), and affixing the at least one fixation ribbon onto a first portion of a first planar surface of a surgical mesh, the first planar surface defined by an area, and the first portion comprising less than the area.

## Description

### FIELD

The present technology is generally related to self-gripping material for use in a surgical mesh for hernia repair.

### BACKGROUND

Prosthetic meshes may be used during both laparoscopic and open surgery for repair of many types of defects and injuries. For example, surgical meshes are commonly used in the repair of hernias. The meshes may be used to provide support to surrounding tissue, as well as to supplement standard suturing.

During the repair of soft tissue hernias, clinicians often fit a prosthesis to the site of the hernia repair, and more particularly the abdominal wall at the hernia site. For the prosthesis, textile meshes are often used. For example, a hernia prosthesis may be a biodegradable or non-biodegradable woven or knitted material used during hernia repair surgeries to increase the strength and structural integrity of the hernia site and promote tissue healing and repair. During a procedure, the hernia mesh may be inserted through a trocar and then be deployed inside the abdominal cavity. However, the size of the trocar may limit the size and/or thickness of the mesh that can be successfully and efficiently deployed during the procedure. Furthermore, additional functional features, such as fixation elements, incorporated into the surgical mesh may complicate this delivery process as well.

### SUMMARY

The techniques of this disclosure generally relate to a self-gripping surgical mesh for use in a hernia repair.

In one aspect, the present disclosure provides a method for forming a surgical mesh having one or more fixation elements, the method including forming at least one looped strip within a mesh sheet. The mesh sheet includes at least one chain-link filament, at least one looping filament interlinked with the at least one chain-link filament, the looping filament forming a plurality of loops, at least a first inlay filament and a second inlay filament interlinked with the at least one chain-link filament, and at least a first temporary filament and a second temporary filament, the first temporary filament interlinked with the first inlay filament and the second temporary filament interlinked with the second inlay filament. The method further includes processing the mesh sheet to convert one or more of the plurality of loops into the one or more fixation elements, removing at least the first temporary filament and the second temporary filament to form at least one fixation ribbon having the one or more fixation elements, and affixing the at least one fixation ribbon onto a first portion of a first planar surface of the surgical mesh, wherein the first planar surface is defined by an area, and wherein the first portion comprises less than the area.

In another aspect, the disclosure provides a method of forming a surgical mesh having one or more barbs, the method including forming at least one looped strip within a mesh sheet. The mesh sheet includes at least one chain-link filament, at least one looping filament interlinked with the at least one chain-link filament, the looping filament forming a plurality of loops, at least a first inlay filament and a second inlay filament interlinked with the at least one chain-link filament and the at least one looping filament, and at least a first temporary filament and a second temporary filament, the first temporary filament interlinked with the first inlay filament and the second temporary filament interlinked with the second inlay filament. The method further includes heat processing the mesh sheet to convert one or more of the plurality of loops into the one or more barbs, removing at least the first temporary filament and the second temporary filament to form at least one fixation ribbon having the one or more barbs, and embroidering the at least one fixation ribbon onto a first portion of a first planar surface of the surgical mesh, wherein the first planar surface further includes a second portion, and the first portion of the first planar surface comprising a first area less than a second area of the second portion of the first planar surface.

In another aspect, the disclosure provides a method of implanting a surgical mesh at a hernia site including inserting a trocar through at least one access site of an abdominal wall of a patient and deploying a surgical mesh through the trocar. The surgical mesh includes a first planar surface opposite a second planar surface, the first planar surface defined by an area, and a fixation ribbon affixed to the first planar surface of the surgical mesh, the fixation ribbon defined by an area and including a plurality of fixation elements, the fixation ribbon affixed to a portion of the first planar surface, the portion comprising less than the area of the first planar surface. The method further includes securing the surgical mesh at the hernia site through an engagement between the plurality of fixation elements and tissue at the hernia site.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIGS. 1A-1B are images that illustrate an exemplary surgical mesh, in accordance with the embodiments presented herein.
FIG. 2 illustrates an image of an enlarged portion of a surgical mesh, in accordance with embodiments presented herein.
FIGS. 3A-3C are schematics illustrating various steps of forming a fixation ribbon from a mesh sheet, in accordance with embodiments presented herein.
FIGS. 4A-4C are schematics illustrating various steps of forming a fixation ribbon from a sheet of filaments, in accordance with embodiments presented herein.
FIGS. 5A-5C are schematics illustrating various steps of forming a fixation ribbon from a sheet of filaments, in accordance with embodiments presented herein.
FIG. 6A is a flow chart illustrating a method of forming a surgical mesh, in accordance with embodiments presented herein.
FIG. 6B is a flow chart illustrating a method of deploying a surgical mesh having a fixation ribbon affixed thereon, in accordance with embodiments presented herein.

### DETAILED DESCRIPTION

The present technology relates to a self-gripping surgical mesh for use in a hernia repair. More particularly, the self-gripping surgical mesh may be a textile mesh with fixation ribbons (or gripping ribbons) adhered onto the mesh surface in order to provide fixation elements for attaching the surgical mesh to the tissue of the patient.

The abdominal wall is composed of fat and muscles interconnected by fascias that forms a boundary encasing the abdominal cavity and abdominal viscera. The abdominal wall protects the internal organs, provides core stability, and aids in facilitating movement of the patient. A continuous membrane called the peritoneum lines the abdominal wall and encases the viscera. A hernia occurs when there is a break or an opening in the abdominal wall such that a portion of the peritoneum protrudes through the break. The protruding peritoneum can cause increased weakening of the abdominal tissue around the opening, which can cause pain, inflammation, vomiting, and various other patient symptoms.

Laparoscopic surgical procedures are often employed in the repair of a hernia. More particularly, to repair the hernia, a surgical mesh is often implanted at the hernia site in order to replace, support, and/or strengthen the weakened abdominal tissue. The mesh may be arranged over, under, or within the hernia site. As such, the mesh must exhibit high mechanical performance in order to properly repair the abdominal wall. Hernia and other surgical meshes may be knitted from biocompatible filaments, which may be biodegradable, non-biodegradable, or combinations thereof. Similar to other textiles, the basic structure of a surgical mesh consists of "courses" and "wales," where courses refer to the rows of fibers running width-wise and the wales refer to the columns of fibers running length-wise. When the wales are perpendicular to the course of fibers, this is referred to as "weft knitting"; and when the courses and wales are roughly parallel to the direction of the fibers, this is referred to as "warp knitting." In non-limiting examples, surgical meshes, such as hernia meshes, are often formed by "warp" knitting"; however, various other methods of forming surgical meshes, including weaving or "weft" knitting, may be employed.

As previously described, the surgical mesh may be formed from biocompatible materials. In some embodiments, the surgical mesh may be formed from non-bioabsorbable materials. In these embodiments, the mesh may be formed of polypropylene. Other suitable non-bioabsorbable materials include, without limitation: polyolefins, such as polyethylene, copolymers of polyethylene and polypropylene, and blends of polyethylene and polypropylene. Other non-bioabsorbable materials which may be utilized include polyesters such as polyethylene terephthalate (PET), polyamides, aramides, expanded polytetrafluoroethylene, polyurethane, polyvinylidene, difluoride (PVDF), polybutester, copper alloy, silver alloy, platinum, medical grade stainless steels such as 316 L medical grade stainless steel, combinations thereof, and the like. Examples of commercially available polypropylene-based textile supports which may be utilized include those sold under the brand name PARIETENE^{™} from Sofradim.

In further embodiments, the surgical mesh may be formed from bioabsorbable materials. In these embodiments, the surgical mesh may have a composition including lactide and trimethylene carbonate (TMC). Other suitable absorbable materials include, without limitation: trimethylene, carbonate, caprolactone, dioxanone, glycolic acid, lactic acid, glycolide, lactide, homopolymers thereof, copolymers thereof, and combinations thereof. Specific absorbable materials which may be suitable include, for example, chitosan, cellulose, oxidized cellulose, combinations thereof, and the like.

Surgical meshes may be described by their physical and mechanical properties. The physical properties of the mesh can include, but are not limited to, length, width, surface density, pore size, thickness, and bending rigidity. The mechanical properties of the mesh can include, but are not limited to, ball-burst strength, uniaxial tensile strength, and suture pull-out strength. In aspects, by altering the materials used and/or knitting or weaving patterns, the physical and/or mechanical properties of the mesh can be significantly changed.

Furthermore, the physical structure of a surgical mesh may be defined by a shape, including rectangular, oval, circular, or square shapes, for example. In other instances, the surgical mesh may have a different shape based on a clinician's and/or a patient's need. Moreover, a surgical mesh may be defined by edges, or sides, interconnecting a first planar surface and an opposing second planar surface. In aspects, the edges may be defined by the thickness of the mesh, and the first planar surface and the second planar surface may be defined by the length and width of the mesh.

In some embodiments, and as will be described further herein, the first planar surface and/or the second planar surface of the mesh may include fixation (or gripping) elements for affixing the mesh to tissue at the hernia site. It can be advantageous to add gripping or fixation elements onto the surgical or hernia mesh in order to support the adherence of the surgical mesh onto the abdominal tissue. More particularly, tissue fixation elements may include barbs, spikes, t-shaped prongs, or other grippers, for anchoring the surgical mesh into the tissue and maintaining the surgical mesh in a position spanning the entirety of the weakened tissue at the hernia site. For example, the tissue fixation elements may be formed from a looping filament that is composed of one or more biocompatible materials, including bioabsorbable materials, non-bioabsorbable materials, or combinations thereof. For example, the looping filament forming the tissue fixation elements may be formed of any of the materials described previously with reference to the surgical mesh. In some embodiments, the surgical mesh is formed of the same material as the tissue fixation elements. In other embodiments, the surgical mesh may be formed of different materials than the tissue fixation elements.

Furthermore, the tissue fixation elements may include a plurality of barbs, spikes, t-shaped prongs, or other grippers. The fixation elements may be formed using a variety of methods, including but not limited to, injection molding, stamping, etching, cutting, laser treatments, ultrasonic treatments, heat processing, and/or any other applicable method known in the industry. For example, a sheet of surgical mesh may be knitted to include a plurality of loops extending from at least one of the first planar surface or the second planar surface. The plurality of loops may then be processed into the plurality of fixation elements by passing the sheet of surgical mesh having the plurality of loops over a cylinder having an electrical heating resistor. As the mesh sheet is passed over the heated cylinder, each loop of the plurality of loops may be melted or otherwise cut or deformed to create a barb, spike, t-shaped prong, or other gripping structure. While this non-limiting example provides one method of creating a plurality of fixation elements, various other processing methods may be incorporated in order to form the plurality of fixation elements. Additionally, whereas the plurality of loops may be formed on either or both of the first planar surface or the second planar surface of a mesh sheet, non-limiting examples herein will describe forming and processing the plurality of loops on one side (e.g., one planar surface) of the mesh sheet.

Once formed, the plurality of tissue fixation elements may be oriented in a variety of different directions relative to the surgical mesh. For example, the tissue fixation elements may be arranged to extend approximately perpendicular with respect to the planar surface of the surgical mesh. In other examples, the tissue fixation elements may extend from approximately 75 degrees to approximately 105 degrees relative to the planar surface of the mesh. In still other instances, the plurality of tissue fixation elements may be oriented between approximately 25 degrees to approximately 105 degrees relative to the planar surface of the surgical mesh.

While the tissue fixation elements may provide the benefit of facilitating the anchoring of the hernia mesh into the tissue of the patient around the hernia site, tissue fixation elements may increase the size of the hernia mesh. For example, the tissue fixation elements may increase the thickness (or the surface density) or decrease the flexibility of the surgical mesh. For example, when the plurality of loops incorporated into the knitted structure of the mesh are processed into the plurality of fixation elements, the fixation elements may extend over a substantial surface of the surgical mesh, thereby greatly increasing the overall thickness of the surgical mesh. As will be described further, this can complicate the process of inserting the hernia mesh through a surgical access device such as a trocar. It is advantageous to have the tissue fixation elements adhered to the surgical mesh, but the surgical mesh needs to maintain the ability to fold, roll, or otherwise be shaped by the clinician such that the mesh can be successfully deployed through a trocar to the desired location within the patient. Moreover, with the increasing popularity of robotic laparoscopic procedures that can be conducted through smaller surgical incisions, trocar dimensions are becoming increasingly smaller.

For example, during laparoscopic repair of a hernia, the trocar, which is a surgical device fitted with a cannula, may be inserted into a surgical access site in the abdominal wall in order to insert various tools into a body cavity of a patient for treating the hernia. Once access to the hernia site is established, the clinician may insert tools through the trocar in order to reposition the herniated tissue back into place within the patient and repair the tissue surrounding the hernia site. After repositioning of the tissue, the clinician may then insert a surgical mesh through the trocar and place it over or within the hernia site in order to provide support and strengthen the hernia site. The dimensions of the trocar may vary, for example, having an average port size (e.g., diameter) of approximately 15 mm for traditional laparoscopic procedures and approximately 8mm for robotic laparoscopic procedures. With such small openings, it can be difficult to insert surgical meshes through the trocar and into the body cavity, particularly for meshes having increased thicknesses and/or increased surface densities due to the integration of tissue fixation elements (e.g., tissue gripping elements). High trocar insertion forces can increase the risk of tearing or deforming the surgical mesh, injuring the patient, and/or causing mesh deployment difficulties for the clinician.

Accordingly, while it is advantageous to optimize the mechanical properties of the surgical mesh to promote adherence to the hernia site, the corresponding physical properties must facilitate insertion of the hernia mesh into small-diameter trocars. In other words, while inclusion of the tissue fixation elements promotes favorable surgical outcomes for hernia repair, the interrelated physical properties should also be optimized (e.g., high flexibility and minimized thickness and/or surface density) for deployment through small-diameter trocars during the surgical procedure.

Thus, the embodiments described herein provide an advantageous method of configuring a surgical mesh with tissue fixation elements while maintaining the physical properties of the mesh within a range acceptable for small-trocar insertion. That is, the resulting surgical mesh may have a relatively small size (i.e., mass, thickness, surface density, etc.) and high flexibility to facilitate insertion through small-diameter trocars, while still incorporating fixation elements, aiding in the positioning and stability of surgical meshes in laparoscopic procedures.

More particularly, the embodiments described herein provide a surgical mesh (e.g., hernia mesh) comprising a first planar surface opposite a second planar surface interconnected by edges corresponding to a thickness of the surgical mesh. The surgical mesh may be composed of any of the materials described above with reference to the biocompatible filament materials. In aspects, the surgical mesh includes a fixation ribbon affixed to at least a first planar surface of the mesh. In aspects, a fixation ribbon is composed of at least one chain-link filament (e.g., chain-link filaments 308), at least one inlay filament (e.g., inlay filaments 306), and at least one looping filament (e.g., looping filament 314). The looping filament may be processed to form a plurality of fixation elements, which may be the same as or similar to the tissue fixation elements described previously. For example, the plurality of fixation elements may include a plurality of barbs, spikes, t-shaped prongs, or combinations thereof, for example, extending along the looping filament. In some instances, the fixation ribbon may include a plurality of looping filaments corresponding to a plurality of columns of fixation elements arranged in the fixation ribbon. Similarly, there may be a plurality of base filaments (e.g., chain-link filaments and/or inlay filaments) providing a structural framework for each fixation ribbon. In some embodiments, the width of the fixation ribbon may have a value of between approximately 0.1 mm to approximately2.0 cm. However, various other values for the width may be incorporated. For example, the width of the fixation ribbon may be adjusted based on the type of fixation and/or embroidery used to affix the fixation ribbon onto the surgical mesh.

As described above, one or more fixation ribbons having fixation elements may be affixed to the surgical mesh, and more particularly, may be affixed to a first portion of the planar surface of the surgical mesh in a predetermined pattern for maintaining the positioning of the hernia mesh at the hernia site. In contrast, one or more fixation ribbons may not be affixed to a second portion of the planar surface of the surgical mesh, thereby minimizing an increased thickness, or surface density, due to the incorporation of the fixation elements. This allows the flexibility and size of the surgical mesh to be maintained within the limits imposed by small-diameter trocars. As should be appreciated, one or more fixation ribbons may be additionally or alternatively affixed to a first portion of the second planar surface, while yet optimizing placement of fixation elements and minimizing an overall thickness and/or surface density of the surgical mesh.

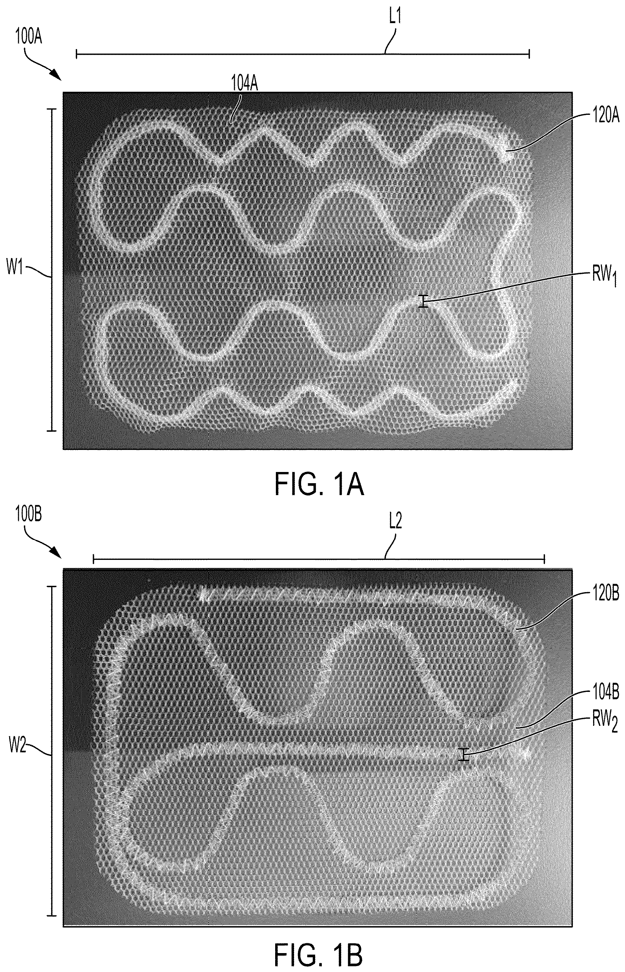

For example, FIGS. 1A and 1B illustrate two exemplary patterns of a fixation ribbon affixed to a surgical mesh. More particularly, FIG. 1A illustrates a first embodiment of a first surgical mesh 100A having a first fixation ribbon 120A affixed thereon. In some instances, the first fixation ribbon 120A may be affixed through embroidery to the first surgical mesh 100A; however, various other methods of affixing the first fixation ribbon 120A to the first surgical mesh 100A may be incorporated. For example, the first fixation ribbon 120A may be adhered to the first surgical mesh 100A (e.g., via a glue or other adhesive), sutured onto the surgical mesh 100A, or otherwise affixed onto the first surgical mesh 100A through other applicable methods, either now known or later developed.

As illustrated, the first fixation ribbon 120A is associated with a first ribbon width, RW₁, and is affixed to a first planar surface 104A of the first surgical mesh 100A. In this example, the first fixation ribbon 120A is applied in a first pattern that winds back and forth over a first portion of the first planar surface 104A but does not entirely cover the first surgical mesh 100A. More particularly, FIG. 1A illustrates the first pattern defined by the first fixation ribbon 120A being arranged with more coverage over a central area of the first planar surface 104A and less coverage near the edges of first planar surface 104A. That is, at least a second portion of the first planar surface 104A remains free of fixation elements. For example, the first planar surface 104A may be defined by a length L1 and a width W1 further defining a two-dimensional surface area of the first planar surface 104A. In embodiments, when the fixation ribbon 120 is affixed to the first portion of the first planar surface 104A, the first portion is defined by an area, such as a two-dimensional surface area, that is less than the surface area of the first planar surface 104A. Furthermore, in some instances, the area of the first portion of first planar surface 104A is less than an area defined by the second portion that remains free of fixation elements. Since the first fixation ribbon 120A, including tissue fixation elements, is affixed only to the first portion of the first planar surface 104A, only the first portion of the first surgical mesh 100A will exhibit an increased thickness (or surface density) due to the first fixation ribbon 120A. Thus, the first surgical mesh 100A includes the fixation elements to support fixation of the first surgical mesh 100A to the herniated tissue, while also maintaining flexibility and a minimal increase in mass (e.g., attributable to increased thickness and/or surface density) of the first surgical mesh 100A.

FIG. 1B illustrates a second embodiment of a second surgical mesh 100B defined by a second planar surface 104B having a second fixation ribbon 120B affixed thereto. As illustrated, the second planar surface 104B is associated with a second length L2 and a second width W2 defining a surface area (for example, a two-dimensional surface area) of the second planar surface 104B. Furthermore, in this example, the second fixation ribbon 120B is associated with a second ribbon width, RW₂, and is applied in a second pattern that winds around and over a first portion of the second planar surface 104B but does not entirely cover the second surgical mesh 100B. In other words, an area defined by the second fixation ribbon 120B may be less than the surface area defined by the second planar surface 104B. In aspects, second ribbon width, RW₂, of second fixation ribbon 120B may be the same as, less than, or greater than the first ribbon width, RW₁, of the first fixation ribbon 120A. As illustrated, the second pattern defined by the second fixation ribbon 120B is arranged with more coverage near the edges of the second planar surface 104B and less coverage over the central area of the second surgical mesh 100B.

As should be appreciated, the first and second patterns illustrated herein are not intended to be limiting, and any of a variety of predetermined patterns that incorporate the benefits of adding fixation elements without unduly increasing the mass of a surgical mesh 100A-100B are contemplated herein. In some embodiments, the pattern may be chosen based on the intended type of surgical procedure (e.g., laparoscopic, robotic, etc.) and/or use of the surgical mesh (e.g., hernia repair, pelvic prolapse repair, reconstructive surgery, vaginal wall reconstruction, etc.). For example, the pattern may be optimized to aid in folding and/or rolling the surgical mesh 100A-100B to facilitate insertion of the surgical mesh 100A-100B into a trocar. In other embodiments, the pattern of applying the fixation ribbon 120 on the first planar surface 104 may be chosen based on the intended application of the surgical mesh 100A-100B, e.g., to promote conformity to various anatomical sites. Furthermore, the amount and/or placement of the fixation ribbon 120 onto the first planar surface 104 may be adjusted based on a size of the trocar intended to be used for the procedure (e.g., 15mm, 8mm, etc.). Furthermore, in the embodiments shown herein, the fixation ribbons 120 are affixed to the first planar surface 104 as continuous structures. However, in other instances, fixation ribbons 120 may be affixed to the first planar surface 104 in multiple discontinuous segments.

In embodiments illustrated by FIGS. 1A and 1B, the surgical mesh 100A-100B may be composed of a biocompatible material that is bioabsorbable, such as lactide and trimethylene carbonate (TMC). Other suitable bioabsorbable materials include, without limitation: trimethylene, carbonate, caprolactone, dioxanone, glycolic acid, lactic acid, glycolide, lactide, homopolymers thereof, copolymers thereof, and combinations thereof. Specific absorbable materials which may be suitable include, for example, chitosan, cellulose, oxidized cellulose, combinations thereof, and the like. However, any bioabsorbable and biocompatible material may be incorporated for use. In other embodiments, the surgical mesh 100A-100B may be composed of a biocompatible material that is non-bioabsorbable. In some embodiments, this may include polypropylene. Other suitable non-bioabsorbable materials include, without limitation: polyolefins, such as polyethylene, copolymers of polyethylene and polypropylene, and blends of polyethylene and polypropylene. Other non-bioabsorbable materials which may be utilized include polyesters such as polyethylene terephthalate (PET), polyamides, aramides, expanded polytetrafluoroethylene, polyurethane, polyvinylidene, difluoride (PVDF), polybutester, copper alloy, silver alloy, platinum, medical grade stainless steels such as 316 L medical grade stainless steel, combinations thereof, and the like. Examples of commercially available polypropylene-based textile supports which may be utilized include those sold under the brand name PARIETENE^{™} from Sofradim. However, in embodiments, any biocompatible textile void of gripping elements may be utilized for the surgical mesh 100A-100B.

As will be described further, each fixation ribbon 120 may include a plurality of fixation elements and may be formed of the same or different materials than the surgical mesh 100A-100B, including any of the biocompatible, bioabsorbable or non-bioabsorbable materials described above, or combinations thereof. The fixation elements of the fixation ribbon 120 allow for the surgical mesh 100A-100B to grip onto tissue, e.g., the abdominal tissue at a hernia site, and increase the security and stability of the surgical mesh 100A-100B during and after surgical positioning.

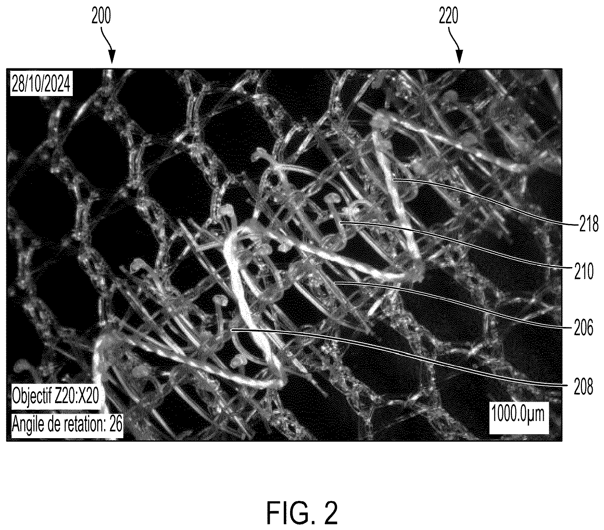

FIG. 2 illustrates a microscopical image (x20) of an enlarged portion of a surgical mesh 200. More specifically, the image of FIG. 2 illustrates a portion of the surgical mesh 200 including a fixation ribbon 220 embroidered thereon by at least one embroidery stitch 218. As illustrated, the fixation ribbon 220 may include a first filament 206, a second filament 208 and a plurality of fixation elements 210 secured to the second filament 208. The plurality of fixation elements 210 are portions of a looping filament (discussed further below) that have been processed to define barbs, loops, spikes, or other features that are configured to grip the tissue once the surgical mesh 200 is implanted into the patient. As illustrated, and as will be described further herein, the fixation elements 210 may be oriented in more than one direction or may be multi-directional along the fixation ribbon 220.

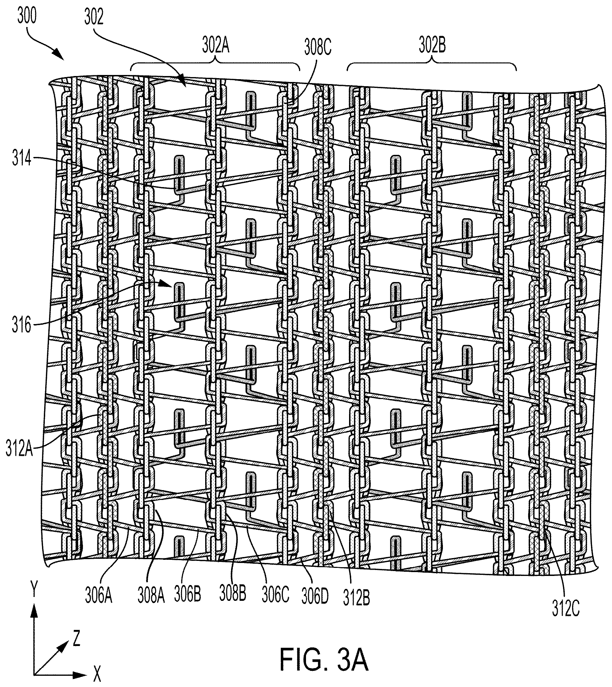

FIGS. 3A-3C are schematics illustrating various components of forming a fixation ribbon (e.g., fixation ribbon 120, 220). For example, FIG. 3A illustrates a mesh sheet 300, which is formed from a plurality of filaments (or yarns) at a first step of processing. As previously described, the mesh sheet 300 may be a knitted sheet of filaments, for example, a warp knit structure formed on a warp knitting machine such as the tricot or Raschel type. However, unlike traditional warp knitted meshes, mesh sheet 300 may include a plurality of looped strips 302 (e.g., looped strips 302A-302B) linked together by temporary filaments 312 (e.g., temporary filaments 312A-312C). In some examples, the temporary filaments 312 may be chain-link filaments.

In aspects, each looped strip 302 may be formed of one or more permanent chain-link filaments 308 (e.g., chain-link filaments 308A-308C), and a looping filament 314 secured by the permanent chain-link filaments 308 and forming a plurality of loops 316. The plurality of loops 316 may be formed by pulling the looping filament 314 during the knitting process. For example, each of the plurality of loops 316 may generally be formed by feeding a needle through a looped yarn with a free end, wrapping the free end around the needle, securing the loop, and subsequently clearing the needle from the looped yarn. Each looped strip 302 may further include a set of inlay filaments 306 (e.g., inlay filaments 306A-306D) that link the permanent chain-link filaments 308 with one another and with the temporary filaments 312.

As illustrated, the temporary chain-link filament 312A-C define longitudinal boundaries for the first looped strip 302A and the second looped strip 302B, which will ultimately form the fixation ribbons (e.g., fixation ribbons 120A-120B, 220). In other words, the number and placement of the temporary filaments 312 dictate a width of each looped strip 302 as well as the number of looped strips 302 within the mesh sheet 300. Thus, during the knitting process, the temporary filaments 312 crosslinked through the inlay filaments 306 dictate how many looped strips 302, and thus fixation ribbons, are formed from the mesh sheet 300.

With further reference to FIG. 3A, the knit structure of the mesh sheet 300 will be described further. The mesh sheet 300 includes at least two looped strips extending therethrough, illustratively a first strip 302A and a second strip 302B. As illustrated, each looped strip 302 includes a set of chain-link filaments 308 (e.g., three chain-link filaments 308A-308C), a looping filament 314, and a set of inlay filaments 306 (e.g., four inlay filaments 306A-306D). As should be appreciated, the knit structure of FIG. 3A is provided for illustrative purposes and other knit structures are contemplated. For example, as discussed further below, a looped strip 302 may include a single chain-link filament 308, a looping filament 314, and two inlay filaments 306. However, in other embodiments, a looped strip 302 may include any number of chain-link filaments 308. For example, the looped strip 302 may include two, three, four or more chain-link filaments 308. The number of chain-link filaments 308 may be decided based on the desired width and/or function of the surgical mesh once formed.

In some embodiments, the chain-link filaments 308, inlay filaments 306, and looping filaments 314 may be composed of a biocompatible, bioabsorbable or non-bioabsorbable materials. For example, the filaments may be composed of bioabsorbable materials such as lactide and trimethylene carbonate (TMC). Other suitable bioabsorbable materials include, without limitation: trimethylene caprolactone, dioxanone, glycolic acid, lactic acid, glycolide, homopolymers thereof, copolymers thereof, and combinations thereof. Specific absorbable materials which may be suitable include, for example, chitosan, cellulose, oxidized cellulose, combinations thereof, and the like. In other embodiments, the filaments may be composed of a biocompatible, non-absorbable materials, such as polypropylene. Other suitable non-bioabsorbable materials include, without limitation: polyolefins, such as polyethylene, copolymers of polyethylene and polypropylene, and blends of polyethylene and polypropylene. Other non-bioabsorbable materials which may be utilized include polyesters such as polyethylene terephthalate (PET), polyamides, aramides, expanded polytetrafluoroethylene, polyurethane, polyvinylidene, difluoride (PVDF), polybutester, copper alloy, silver alloy, platinum, medical grade stainless steels such as 316 L medical grade stainless steel, combinations thereof, and the like. Examples of commercially available polypropylene-based textile supports which may be utilized include those sold under the brand name PARIETENE^{™} from Sofradim. However, the above materials are intended as examples and other applicable materials may be contemplated and incorporated.

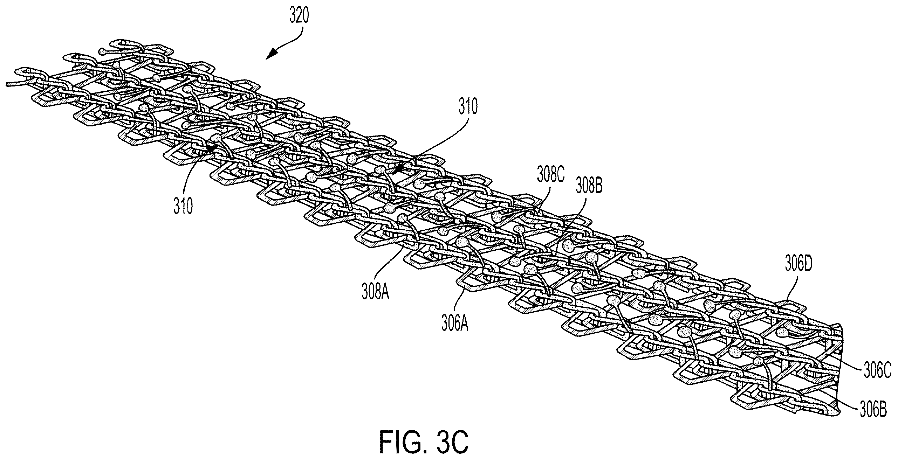

As illustrated in FIG. 3A, the first looped strip 302A includes a first permanent chain-link filament 308A linked with a first inlay filament 306A and a second inlay filament 306B. The first looped strip 302A further includes a second permanent chain-link filament 308B linked with the second inlay filament 306B and a third inlay filament 306C. First looped strip 302A further includes a third permanent chain-link filament 308C linked with the third inlay filament 306C and a fourth inlay filament 306D. Furthermore, the first looped strip 302A includes a looping filament 314 interlinked with permanent chain-link filaments 308A, 308B, and 308C. As illustrated, the looping filament 314 includes a plurality of loops 316 defined therein. As illustrated, the plurality of loops 316 may extend along a direction approximately perpendicular to longitudinal Y axis along the Z axis with respect to the mesh sheet 300. The orientation of the plurality of loops 316 is an indication of the directionality of a plurality of fixation elements that will be formed from the loops 316 in later manufacturing steps, as will be described further with reference to FIG. 3C. Furthermore, with reference still to FIG. 3A, the second looped strip 302B is substantially the same as first looped strip 302A. For example, the second looped strip 302B may also include three permanent chain-link filaments, four inlay filaments, and a looping filament. In aspects, the configuration of the looped strips within the mesh may be consistent, that is, all looped strips may have the same number of chain-link filaments, looping filaments, and inlay filaments. However, in other embodiments, the looped strips 302 may have different configurations within the mesh sheet, divided by temporary filaments.

In some embodiments, mesh sheet 300 may undergo heat processing through the use of an electrical heating resistor. More particularly, mesh sheet 300, including a plurality of looped strips 302, may be passed over a heated cylinder. The looped strips 302 including the plurality of loops 316 may be pressed flat onto the cylinder by two pairs of rollers that are arranged downstream and upstream of the heated cylinder. Once the looped strips 302 are passed over the cylinder, at least a subset of the plurality of loops 316 may be melted or otherwise deformed to create a plurality of barbs 310 (see FIG. 3C). However, various other processing methods may be incorporated in order to process the plurality of loops 316 into the plurality of barbs 310 (e.g., fixation elements).

With reference still to FIG. 3A, the first and second looped strips 302A, 302B are temporarily linked with temporary filaments 312. For example, a first temporary filament 312A is interlinked on a first side of the first looped strip 302A and a second temporary filament 312B arranged on a second side of the first looped strip 302A. Furthermore, the second temporary filament 312B is positioned on a first side of second looped strip 302B and a third temporary filament 312C is positioned on a second side of the second looped strip 302B. As will be described further herein, following heat processing of the mesh sheet 300, removal of the temporary filaments 312 allows for the separation of the looped strips 302 into individual fixation ribbons. In other words, the temporary filaments 312 secure the positioning of the looped strips 302 within the mesh sheet 300 during heat processing. As the temporary filaments 312 are arranged adjacent either side of each looped strip 302, the positioning of the temporary filaments 312 may define the width of each looped strip 302 and the number of looped strips 302 arranged laterally across the mesh, which are later separated from one another to form the independent fixation ribbons.

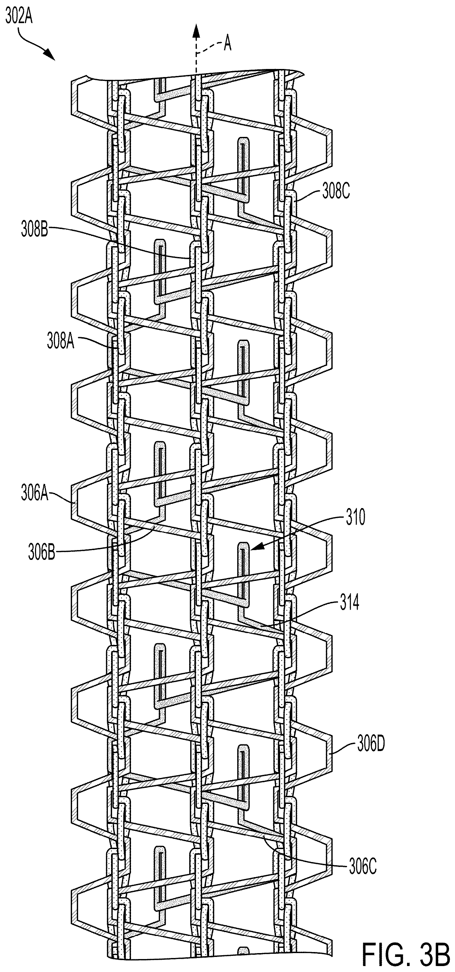

For example, FIG. 3B illustrates a portion of the mesh sheet 300 of FIG. 3A, after the temporary filaments 312 (FIG. 3A) have been removed. After removal of the temporary filaments 312, each of the looped strips 302A, 302B may be separate or distinct from one another. For example, FIG. 3B illustrates the first looped strip 302A after separation from second looped strip 302B. In these instances, the first permanent chain-link filament 308A remains linked with the first inlay filament 306A and the second inlay filament 306B, the second permanent chain-link filament 308B remains linked with the second inlay filament 306B and the third inlay filament 306C, and the third permanent chain-link filament 308C remains linked with the third inlay filament 306C and the fourth inlay filament 306D.

Furthermore, the ability of the looped strips 302 to be separated through the removal of the temporary filaments 312 facilitates the separation of the looped strips 302 without requiring the strip filaments along the edges to be directly cut or severed. In other words, the temporary filaments 312 can be pulled out of the mesh sheet 300, facilitating separation of the looped strips 302 while maintaining the finished edges of the looped strips 302. For example, as illustrated, the inlay filaments 306 are defined by continuous and enclosed filaments throughout the linkages with permanent chain-link filaments 308. Additionally, in FIG. 3B, the first looped strip 302A further includes a looping filament 314 that forms the plurality of loops 316, the looping filament 314 maintaining linkages with at least the permanent chain-link filaments 308A-308C and/or the inlay filaments 306A-306D.

FIG. 3C illustrates fixation ribbon 320, which corresponds to the first looped strip 302A after undergoing heat processing and being separated from the second looped strip 302B by removing the temporary filaments 312A-312B (see FIG. 3A). More particularly, during the heat processing, the plurality of loops 316 (FIG. 3A) are processed to become a plurality of barbs 310 illustrated in FIG. 3C. In some embodiments, mesh sheet 300 (FIG. 3A) may undergo heat processing through the use of an electrical heating resistor. More particularly, as described above, the looped strips 302 having the plurality of loops 316, may be passed over a heated cylinder. The looped strips 302 may be pressed flat onto the heated cylinder by two pairs of rollers that are arranged downstream and upstream of the heated cylinder. Once the looped strips 302 are passed over the heated cylinder, at least a subset of the plurality of loops 316 of the looped strips 302 can be melted, cut, or otherwise deformed to create the plurality of barbs 310. However, various other processing methods may be incorporated in order to process the plurality of loops 316 into the plurality of barbs 310.

As previously described, the plurality of barbs 310 are configured to be tissue engaging or gripping features (or self-gripping features), such that the plurality of barbs 310 work to secure the surgical mesh 100A-100B (FIGS. 1A-1B) to the tissue of the patient after hernia repair. In other words, the plurality of barbs 310 are configured for fixing the surgical mesh 100A-100B (FIGS. 1A-1B) to the weakened tissue around the hernia site, such as the abdominal wall.

While the fixation ribbon 320 is described as being heat processed before the removal of the temporary filaments 312, in some embodiments, the looped strips 302 may be heat processed after the removal of the temporary filaments 312. In other words, the individual looped strips 302 may be processed such that the plurality of loops 316 (FIG. 3A) are converted into a plurality of fixation elements, or a plurality of barbs 310, after the removal of the temporary filaments 312 and separation of the mesh sheet 300 into a plurality of looped strips 302. In this case, the looped strips 302 may be treated by a passing them over a smaller heated cylinder or passing them through a heated cylinder, for example.

As described above, once prepared and separated from the mesh sheet 300, the fixation ribbon 320 may then be embroidered, or otherwise affixed or adhered onto a planar surface of a surgical mesh, as discussed with reference to FIGs. 1A-1B, in order to form a self-gripping surgical mesh while minimizing added thickness, surface density, and/or stiffness.

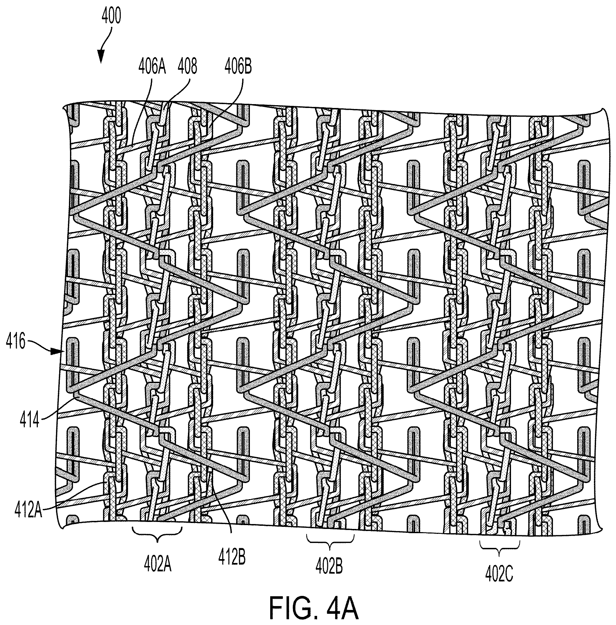

FIGs. 4A-4C are schematics showing various steps of an exemplary formation process for producing an additional embodiment of a fixation ribbon. For example, FIG. 4A illustrates a mesh sheet 400 including at least three looped strips, illustratively looped strips 402A, 402B, and 402C. The following description will be made primarily with reference to looped strip 402A, however, it may apply to looped strips 402B, 402C as well. For example, looped strip 402A includes a first permanent chain-link filament 408 linked with a first inlay filament 406A and a second inlay filament 406B. Furthermore, a looping filament 414 is linked with the first chain-link filament 408 and extends radially outward from the first chain-link filament 408. As illustrated, the looping filament 414 forms a plurality of loops 416 defined thereby. Furthermore, as illustrated, a first temporary filament 412A is linked with the first inlay filament 406A and a second temporary filament 412B linked with the second inlay filament 406B. As previously described, the temporary filaments 412 secure the looped strips 402 to one another during processing of the mesh sheet 400. As will be illustrated and described further herein, the arrangement of the temporary filaments 412 may define the width of the looped strips 402, and thus the fixation ribbons formed from the looped strips 402. In this way, during the manufacturing of the mesh sheet 400, the incorporation, and more particularly the number of and positioning of the temporary filaments 412 can be chosen based on the desired width of the resulting fixation ribbons.

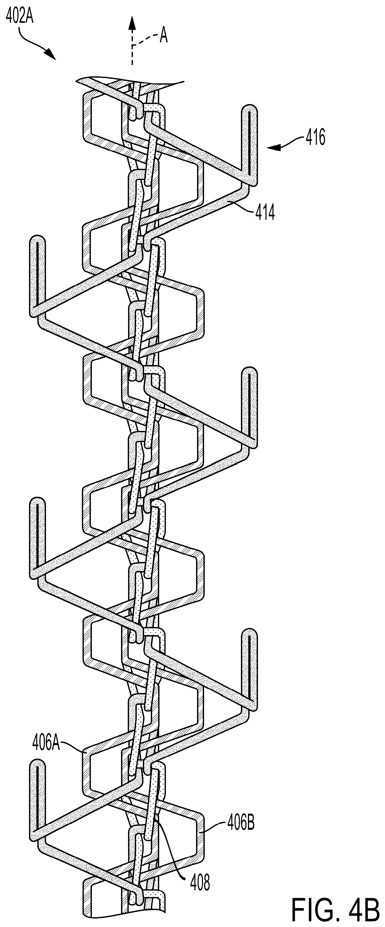

With reference now to FIG. 4B, the looped strip 402A of the mesh sheet 400 (FIG. 4A) is illustrated after removal of the temporary filaments 412A, 412B. In this instance, looped strip 402A is no longer intertwined within the mesh sheet 400 but is instead separated into the singular looped strip 402A. As illustrated, by removing the temporary filaments 412A, 412B, looped strip 402A can be separated from the mesh sheet 400 without requiring cutting and/or severing portions of the first and second inlay filaments 406A, 406B. In other words, the looped strips 402 can have finished edges due to the method of separation through the removal of the temporary filaments 412. Additionally, the looping filament 414 maintains integration with the cross-link filament 408 and the first and second inlay filaments 406A, 406B. In this view, the plurality of loops 416 extend in a direction that is approximately perpendicular to a longitudinal axis A of the looped strip 402A.

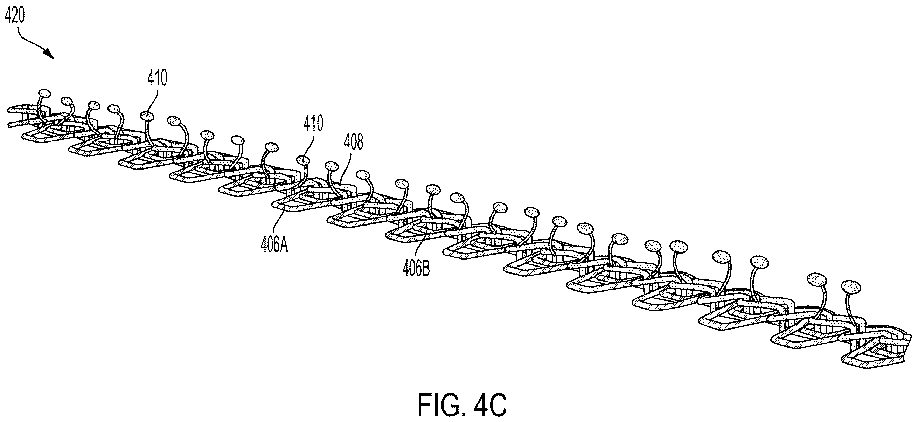

FIG. 4C illustrates a fixation ribbon 420 resulting from the looped strip 402A of FIG. 4B after heat processing is applied and temporary filaments 412 have been removed. In these embodiments, the heat processing facilitates the formation of a plurality of barbs 410 from the previously illustrated plurality of loops 416 of the looped strip 402A (see FIG. 4B). The heat processing method may include the method described with reference to FIGS. 3B-3C. As illustrated in FIG. 4C, the plurality of barbs 410 are oriented such that they extend approximately perpendicularly to longitudinal axis A. However, various other orientations of the plurality of barbs may be incorporated.

As described previously, once prepared as described above, the fixation ribbon 420 may be embroidered, or otherwise affixed or adhered, to a textile surface, such as first planar surface 104A or second planar surface 104B (see FIGS. 1A-1B). In this example, the plurality of barbs 410 may act as fixation elements configured to engage the tissue of the patient. Furthermore, in various other embodiments the structure of the looped strip 402A, and thus the fixation ribbon 420, may vary. For example, the fixation ribbon 420 may include any number of chain-link filaments, inlay filaments and looping filaments for forming various configurations of fixation ribbons with varying width and/or thicknesses.

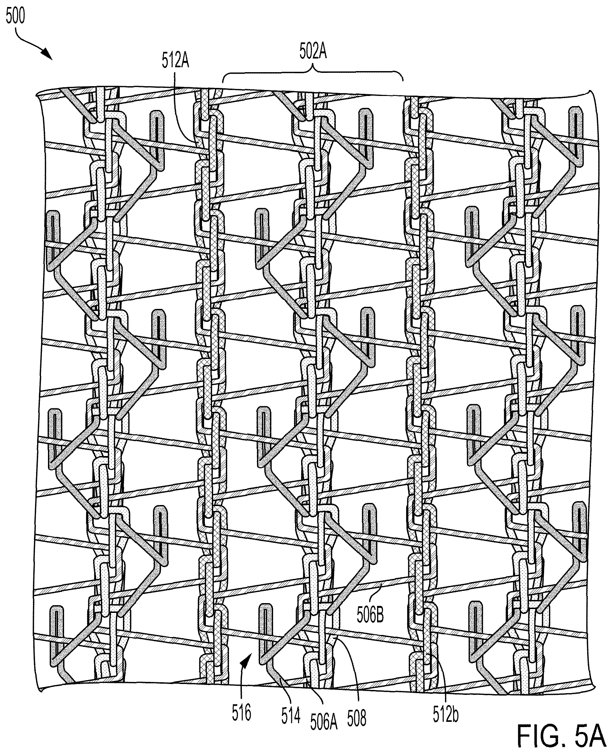

FIGs. 5A-5C illustrate additional schematics of steps of a process for forming an additional embodiment of a forming a fixation ribbon. For example, FIG. 5A illustrates a mesh sheet 500 including at least one looped strip 502 composed of various filaments. While the following description is made with reference a first looped strip 502A, the description may apply to various other looped strips, such as second and third looped strips of the mesh sheet 500. As illustrated, the first looped strip 502A includes a chain-link filament 508 linked with a looping filament 514. The chain-link filament 508 may additionally be linked with a first inlay filament 506A and a second inlay filament 506B. As illustrated, the looping filament 514 forms a plurality of loops 516 extending therefrom. Additionally, in these instances, the plurality of loops 516 extend outward in a direction approximately perpendicular to the looped strip 502A which may dictate the orientation of a plurality of fixation elements that may be formed from the plurality of loops 516, as will be described further herein. In some embodiments, the looped strip 502A is bounded by a first temporary filament 512A linked with the first inlay filament 506A. The looped strip 502A further bounded by a second temporary filament 512B linked with the second inlay filament 506B.

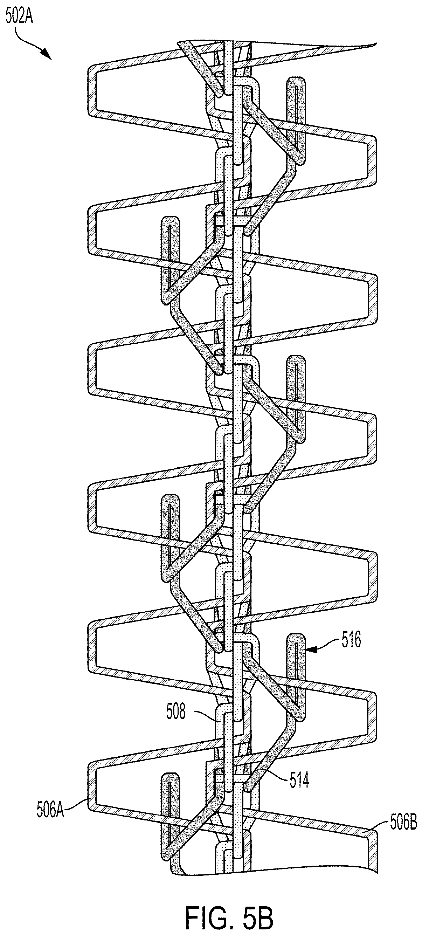

FIG. 5B illustrates the looped strip 502A of FIG. 5A after removal of the temporary filaments 512A, 512B. As illustrated, the first chain-link filament 508 remains linked with the first and second inlay filaments 506A, 506B, and the looping filament 514, which forms the plurality of loops 516.

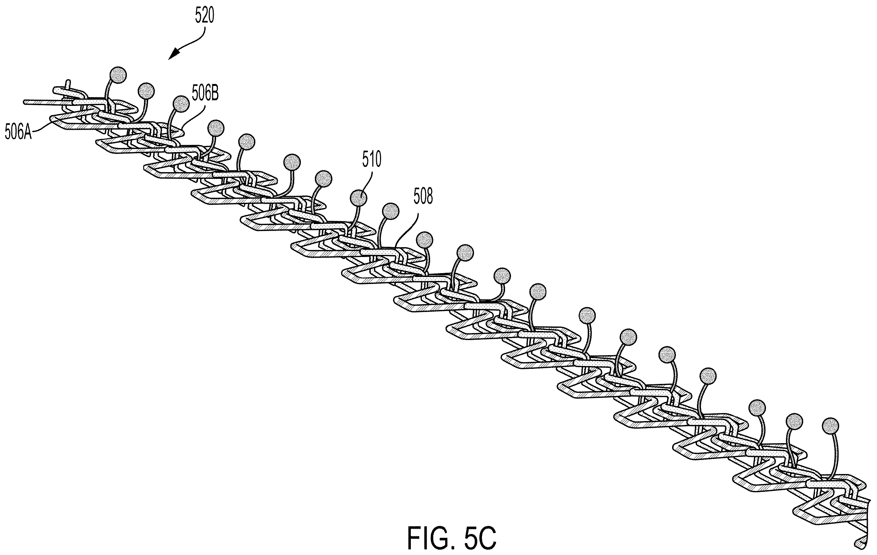

FIG. 5C illustrates the resulting fixation ribbon 520 corresponding to looped strip 502A of FIG. 5B after being processed through the heating process. As illustrated, from the heat processing step, each of the plurality of loops 516 (FIG. 5B) are formed into a plurality of fixation elements, and more particularly a plurality barbs 510. Thus, the fixation ribbon 520 is provided with fixation elements such that after processing fixation ribbon 520 with a textile surface to form a surgical mesh, such as the surgical mesh 100A, 100B (FIGs. 1A-1B), the plurality of barbs 510 facilitate a method of securing the surgical mesh 100A, 100B to the tissue of the patient.

Additionally, while the looped strips described herein with reference to FIGS. 3A-5C all include a single looping filament forming a plurality of loops that are processed into fixation elements (e.g., barbs) during the heat processing of the mesh sheet, the structure of the looped strips may vary. For example, during knitting of the mesh sheet, for example mesh sheet 500 of FIG. 5C, the method may be adjusted to incorporate more or less filaments to increase or decrease the width of the looped strips and to increase or decrease the number of loops (e.g., resulting in fixation elements) that are incorporated into each looped strip. For example, in some embodiments, each looped strip 502 of the mesh sheet 500 may include more than one looping filament 514 such that more loops and, thus, more fixation elements, can be incorporated into the formed fixation ribbon. In some instances, each looped strip 502 may include two looping filaments 514. However, any number of looping filaments 514 may be incorporated. Similarly, the number of chain-link filaments and/or inlay filaments may be increased or decreased depending on the desired composition and size of the fixation ribbon to be formed. This flexibility is facilitated by the ability to incorporate the temporary filaments at the desired locations to define edges of each looped strip 502. Furthermore, a density of fixation elements within the fixation ribbon and the weight of the fixation ribbon, and thus the density of fixation elements within the surgical mesh and the weight of the surgical mesh, can be adjusted during manufacturing processes to achieve the targeted parameters in order to balance the functionality of the surgical mesh with the ability to be inserted through the trocar by the clinician.

The fixation ribbon 520 generated from the above-described process applied to the mesh sheet 500 may then be processed onto a textile surface, such as first planar surface 104A (FIG. 1A) or second planar surface 104B (FIG. 1B) to form a surgical mesh, such as surgical mesh 100A-100B (FIGs. 1A-1B).

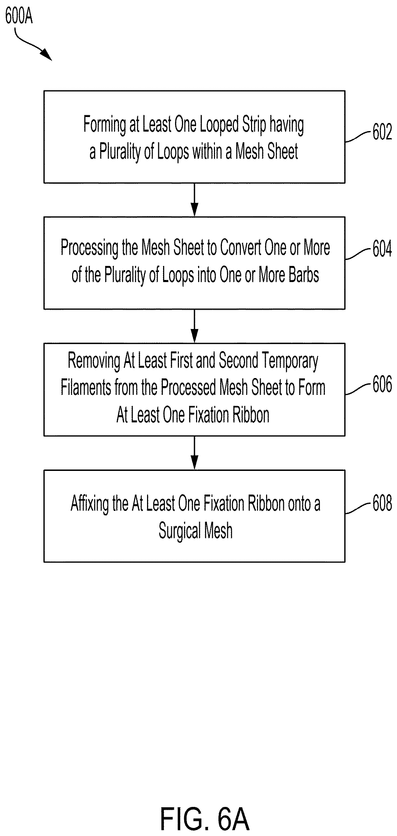

For example, FIG. 6A illustrates a method 600A for forming a surgical mesh, such as surgical mesh 100A, 100B (FIGs. 1A-1B). As illustrated herein, the method 600A will be described largely in the context of the surgical mesh 100A, 100B, and 300 as described in FIGS. 1A, 1B and 3A-3C, however, the method 600A may apply to the formation of surgical mesh 400, 500 described with reference to the embodiments of FIGS. 4A-5C.

As illustrated in FIG. 6A, the method 600A first includes forming at least one looped strip within a mesh sheet at step 602. For example, this may include forming the at least one looped strip 302A within the mesh sheet 300. As described with reference to FIGs. 3A-3C, the looped strip 302A may include at least one chain-link filament 308A, at least one looping filament 314 interlinked with the least one chain-link filament 308A, and the at least one looping filament 314 forming a plurality of loops 316. The looped strip 302A may further include at least the first inlay filament 306A and the second inlay filament 306B interlinked with the first chain-link filament 308A and the at least one looping filament 314. Furthermore, the at least one looped strip 302A includes at least the first temporary filament 312A and the second temporary filament 312B. In these instances, the first temporary filament 312A may be interlinked with the first inlay filament 306A and the second temporary filament 312B may be interlinked with the second inlay filament 306B.

The method 600A may then include step 604 of processing the mesh sheet 300 to convert one or more of the plurality loops 316 into one or more barbs 310. In some embodiments, step 604 may include heat processing the mesh sheet 300, for example through the use of an electrical heating resistor, as previously described.

The method 600A may further include step 606 comprising removing at least the first temporary filament 312A and the second temporary filament 312B to form at least one fixation ribbon (i.e., fixation ribbon 320). In some embodiments, step 606 includes mechanically removing the first and second temporary filaments 312A-B, such as through pulling the first and second temporary filaments 312A-B from the mesh sheet 300.

With reference still to FIG. 6A, the method 600A may then include step 608 of affixing the at least one fixation ribbon 320 onto a surgical mesh, for example the surgical mesh 100A, 100B of FIGs. 1A-1B. In some embodiments, the step 608 may include affixing the at least one fixation ribbon 320 on a first portion of the first planar surface 104A of the surgical mesh 100A. In these instances, the first planar surface 104A is defined by an area, such as the two-dimensional surface area illustrated in FIG. 1A, and the first portion comprises less area than the surface area of the first planar surface 104A. In other words, the first portion of the first planar surface 104A that includes the fixation ribbon 320 affixed thereon, defines an area that is less than the surface area of the first planar surface 104A.

Furthermore, in some instances, step 608 includes embroidering the fixation ribbon 320 onto the first planar surface 104A. In other instances, step 608 may include one of a variety of methods for adhering or otherwise affixing the fixation ribbon 320 onto the first planar surface 104A. Additionally, as described previously herein, affixing the fixation ribbon 320 onto the surgical mesh 100A may include affixing the fixation ribbon 320 in a predetermined pattern. For example, the fixation ribbon 120 may be placed onto the first planar surface 104A in the predetermined pattern shown in FIGs. 1A and 1B. In other instances, the predetermined pattern may include a spherical, helical, linear, stepped, or diagonal pattern. These are provided merely as examples and other applicable patterns may be incorporated depending on the size of the trocar and procedure being implemented.

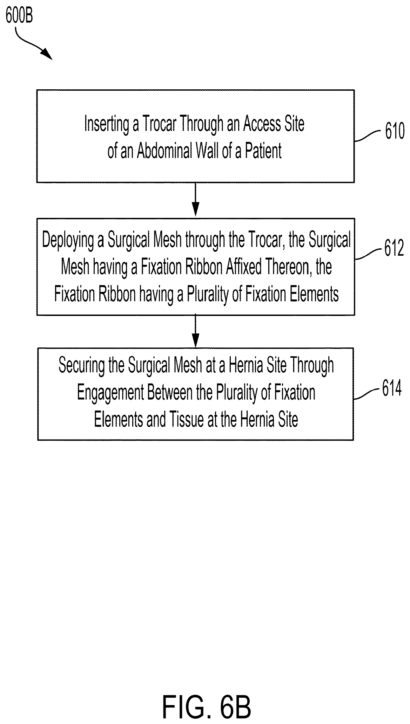

As previously described, the surgical mesh 100 may then be used for securing and/or strengthening a hernia site of the patient as part of a hernia repair procedure. For example, FIG. 6B illustrates a method 600B for implanting a surgical mesh, such as surgical mesh 100A, 100B (FIGs. 1A-1B) at a hernia site. At step 610, the method 600B includes inserting a trocar through an access site of an abdominal wall of a patient. This allows for access to the hernia site to be established. The method 600B further includes step 612 of deploying the surgical mesh 100A, 100B through the trocar. The surgical mesh 100A, 100B includes at least one fixation ribbon (e.g., fixation ribbons 120A, 120B of FIGs. 1A-1B) affixed onto a first planar surface (e.g., the first planar surface 104A of FIG. 1A or the second planar surface 104B of FIG. 1B) of the surgical mesh 100A, 100B. In aspects, since the fixation ribbon may be affixed to a portion of a planar surface of the surgical mesh, self-gripping features may be conferred to the surgical mesh without substantially increasing a thickness or mass of the surgical mesh. In this way, the self-gripping surgical mesh may be inserted through a trocar without an unacceptable insertion force. In aspects, an unacceptable insertion force may cause tearing or other deformation diminishing the operative mechanical function of the surgical mesh. In some cases, the self-gripping surgical mesh may be suitable for insertion through a small-diameter trocar suitable for robotic surgical procedures.

With reference still to FIG. 6B, the method 600B includes step 614 of securing the surgical mesh 100A, 100B at the hernia site through engagement between the plurality of fixation elements (e.g., plurality of barbs 310) and the tissue at the hernia site. In some examples, the surgical mesh 100A, 100B can be fixed around, above, or below the hernia site of the patient. The step 614 of securing the surgical mesh 100A, 100B and more particularly the plurality of barbs 310, with the tissue around the hernia site of the patient ensures that the surgical mesh 100A, 100B maintains the desired positioning during and after the procedure. Moreover, in some cases, the positioning of the fixation ribbon on the surgical mesh, e.g., in a particular pattern or position on the surgical mesh, may facilitate securing the self-gripping surgical mesh at the hernia site.

As previously described herein, the surgical mesh 100A-100B (FIGs. 1A-1B) may be resorbable such that the surgical mesh 100A, 100B is absorbed by the surrounding tissue of the patient, promoting new tissue growth in the site of repair. In these instances, the surgical mesh 100A, 100B diminishes in strength and is not intended to be a long-term repair solution. In other embodiments, the surgical mesh 100A, 100B may be permanent, such that it may remain in the body of the patient indefinitely for continued support to the hernia repair site.

The following describes aspects of the disclosure herein that may be used alone or in combination.

Example 1: A method for forming a surgical mesh having one or more fixation elements, the method comprising: forming at least one looped strip within a mesh sheet, comprising: at least one chain-link filament; at least one looping filament interlinked with the at least one chain-link filament, the looping filament forming a plurality of loops; at least a first inlay filament and a second inlay filament interlinked with the at least one chain-link filament; at least a first temporary filament and a second temporary filament, the first temporary filament interlinked with the first inlay filament and the second temporary filament interlinked with the second inlay filament; processing the mesh sheet to convert one or more of the plurality of loops into the one or more fixation elements; removing at least the first temporary filament and the second temporary filament to form at least one fixation ribbon having the one or more fixation elements; and affixing the at least one fixation ribbon onto a first portion of a first planar surface of the surgical mesh, wherein the first planar surface is defined by an area, and wherein the first portion comprises less than the area.

Example 2: The method of example 1, wherein the fixation ribbon confers a gripping feature on the surgical mesh.

Example 3: The method of example 1 or example 2, wherein the first inlay filament and the second inlay filament define a finished edge of the fixation ribbon.

Example 4: The method of any one of examples 1-3, wherein processing the mesh sheet further comprises heat processing the mesh sheet.

Example 5: The method of any one of examples 1-4, wherein affixing the at least one fixation ribbon includes affixing the at least one fixation ribbon in a predetermined pattern.

Example 6: The method of any one of examples 1-5, wherein the surgical mesh is insertable into a trocar.

Example 7: The method of any one of examples 1-6, wherein the surgical mesh is insertable into a small-diameter trocar.

Example 8: The method of any one of examples 1-7, wherein the surgical mesh is suitable for robotic laparoscopic surgery.

Example 9: The method of any one of examples 1-8, wherein the at least one fixation ribbon is defined by a width, and the width may have a value between approximately 0.1 mm and approximately 2.0 cm.

Example 10: A method of forming a surgical mesh having one or more barbs, the method comprising: forming at least one looped strip within a mesh sheet, comprising: at least one chain-link filament; at least one looping filament interlinked with the at least one chain-link filament, the looping filament forming a plurality of loops; at least a first inlay filament and a second inlay filament interlinked with the at least one chain-link filament and the at least one looping filament; at least a first temporary filament and a second temporary filament, the first temporary filament interlinked with the first inlay filament and the second temporary filament interlinked with the second inlay filament; heat processing the mesh sheet to convert one or more of the plurality of loops into the one or more barbs; removing at least the first temporary filament and the second temporary filament to form at least one fixation ribbon having the one or more barbs; and embroidering the at least one fixation ribbon onto a first portion of a first planar surface of the surgical mesh, wherein the first planar surface further includes a second portion, and wherein the first portion of the first planar surface comprises a first area less than a second area of the second portion of the first planar surface.

Example 11: The method of example 10, wherein the fixation ribbon confers a gripping feature on the surgical mesh.

Example 12: The method of example 10 or example 11, wherein the first inlay filament and the second inlay filament define a finished edge of the fixation ribbon.

Example 13: The method of any one of examples 10-12, wherein embroidering the at least one fixation ribbon onto the first portion of the first planar surface includes embroidering the at least one fixation ribbon in a predetermined pattern.

Example 14: The method of any one of examples 10-13, wherein the surgical mesh is insertable into a trocar.

Example 15: The method of any one of examples 10-14, wherein the surgical mesh is insertable into a small-diameter trocar.

Example 16: The method of any one of examples 10-15, wherein the surgical mesh is suitable for robotic laparoscopic surgery.

Example 17: A method of implanting a surgical mesh at a hernia site, the method including: inserting a trocar through at least one access site of an abdominal wall of a patient; deploying a surgical mesh through the trocar, the surgical mesh including: a first planar surface opposite a second planar surface, the first planar surface defined by an area; a fixation ribbon affixed to the first planar surface of the surgical mesh, the fixation ribbon defined by an area and including a plurality of fixation elements, the fixation ribbon affixed to a portion of the first planar surface, the portion comprising less than the area of the first planar surface; securing the surgical mesh at the hernia site through an engagement between the plurality of fixation elements and tissue at the hernia site.

Example 18: The method of example 17, wherein the fixation ribbon confers a gripping feature on the surgical mesh.

Example 19: The method of example 17 or example 18, wherein the trocar is a small-diameter trocar.

Example 20: The method of any one of examples 17-19, wherein the surgical mesh is suitable for robotic laparoscopic surgery.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

## Claims

1. A method for forming a surgical mesh (100A, 100B) having one or more fixation elements, the method comprising:
forming at least one looped strip within a mesh sheet (300, 400, 500), comprising:
at least one chain-link filament (308, 408, 508);
at least one looping filament (314, 414, 514) interlinked with the at least one chain-link filament, the looping filament forming a plurality of loops;
at least a first inlay filament (306A, 406A, 506A) and a second inlay filament (306B, 406B, 506B) interlinked with the at least one chain-link filament;
at least a first temporary filament (312A, 412A, 512A) and a second temporary filament (312B, 412B, 512B), the first temporary filament interlinked with the first inlay filament and the second temporary filament interlinked with the second inlay filament;
processing the mesh sheet to convert one or more of the plurality of loops into the one or more fixation elements;
removing at least the first temporary filament and the second temporary filament to form at least one fixation ribbon (120, 220, 320, 420, 520) having the one or more fixation elements; and
affixing the at least one fixation ribbon onto a first portion of a first planar surface (104A) of the surgical mesh, wherein the first planar surface is defined by an area, and wherein the first portion comprises less than the area.

2. The method of claim 1, wherein the fixation ribbon confers a gripping feature on the surgical mesh.

3. The method of any of claims 1-2, wherein the first inlay filament (306A, 406A, 506A) and the second inlay filament (306B, 406B, 506B) define a finished edge of the fixation ribbon (120, 220, 320, 420, 520).

4. The method of any of claims 1-3, wherein processing the mesh sheet (300, 400, 500) further comprises heat processing the mesh sheet.

5. The method of any of claims 1-4, wherein affixing the at least one fixation ribbon (120, 220, 320, 420, 520) includes affixing the at least one fixation ribbon in a predetermined pattern.

6. The method of any of claims 1-5, wherein the surgical mesh (100A, 100B) is insertable into one or more of a trocar and a small-diameter trocar; and
wherein, optionally, the surgical mesh is suitable for robotic laparoscopic surgery.

7. The method of any of claims 1-6, wherein the at least one fixation ribbon (120, 220, 320, 420, 520) is defined by a width, and the width may have a value between approximately 0.1 mm and approximately 2.0 cm.

8. A method of forming a surgical mesh (100A, 100B) having one or more barbs, the method comprising:
forming at least one looped strip within a mesh sheet (300, 400, 500), comprising:
at least one chain-link filament (308, 408, 508);
at least one looping filament (314, 414, 514) interlinked with the at least one chain-link filament, the looping filament forming a plurality of loops;
at least a first inlay filament (306A, 406A, 506A) and a second inlay filament (306B, 406B, 506B) interlinked with the at least one chain-link filament and the at least one looping filament;
at least a first temporary filament (312A, 412A, 512A) and a second temporary filament (312B, 412B, 512B), the first temporary filament interlinked with the first inlay filament and the second temporary filament interlinked with the second inlay filament;
heat processing the mesh sheet to convert one or more of the plurality of loops into the one or more barbs;
removing at least the first temporary filament and the second temporary filament to form at least one fixation ribbon (120, 220, 320, 420, 520) having the one or more barbs; and
embroidering the at least one fixation ribbon onto a first portion of a first planar surface (104A) of the surgical mesh, wherein the first planar surface further includes a second portion, and wherein the first portion of the first planar surface comprises a first area less than a second area of the second portion of the first planar surface.

9. The method of claim 8, wherein the fixation ribbon (120, 220, 320, 420, 520) confers a gripping feature on the surgical mesh (100A, 100B).

10. The method of any of claims 8-9, wherein the first inlay filament (306A, 406A, 506A) and the second inlay filament (306B, 406B, 506B) define a finished edge of the fixation ribbon (120, 220, 320, 420, 520).

11. The method of any of claims 8-10, wherein embroidering the at least one fixation ribbon (120, 220, 320, 420, 520) onto the first portion of the first planar surface (104A) includes embroidering the at least one fixation ribbon in a predetermined pattern.

12. The method of any of claims 8-11, wherein the surgical mesh (100A, 100B) is insertable into one or more of a trocar and a small-diameter trocar; and
wherein, optionally, the surgical mesh is suitable for robotic laparoscopic surgery.

13. A method of implanting a surgical mesh (100A, 100B) at a hernia site, the method including:
inserting a trocar through at least one access site of an abdominal wall of a patient;
deploying a surgical mesh through the trocar, the surgical mesh including:
a first planar surface (104A) opposite a second planar surface (104B), the first planar surface defined by an area;
a fixation ribbon (120, 220, 320, 420, 520) affixed to the first planar surface of the surgical mesh, the fixation ribbon defined by an area and including a plurality of fixation elements, the fixation ribbon affixed to a portion of the first planar surface, the portion comprising less than the area of the first planar surface;
securing the surgical mesh at the hernia site through an engagement between the plurality of fixation elements and tissue at the hernia site.

14. The method of claim 13, wherein the fixation ribbon (120, 220, 320, 420, 520) confers a gripping feature on the surgical mesh (100A, 100B).

15. The method of any of claims 13-14, wherein the trocar is a small-diameter trocar; and
wherein, optionally, the surgical mesh (100A, 100B) is suitable for robotic laparoscopic surgery.
